# EUROPEAN PATENT APPLICATION

(11) **EP 4 541 301 A1**
(43) Date of publication of application: **23.04.2025**
(21) Application number: 23204182.2
(22) Date of filing: 17.10.2023
(51) Int. Cl.: A61B 18/02, A61B 18/00, A61B 90/00

(54) **COOLING DEVICE FOR CRYOABLATION CATHETER WITH ENHANCED PERFORMANCE**

(71) Applicant: Cryovasc GmbH, 10119 Berlin (DE)
(72) Inventor: HEINER, Wilfred Peter, 10119 Berlin (DE); KERN, Karoline, 10119 Berlin (DE); MAKOUIE, Nima, 10119 Berlin (DE); KLEMM, Holger, 10119 Berlin (DE)
(74) Representative: Stütz, Jan

(57) **Abstract**

The invention relates to a cooling device for cooling a distal catheter portion of a cryoablation catheter configured for ablation, the cooling device comprising a catheter tube formed with a lumen having a proximal catheter region and a distal catheter region, at least one gas expansion chamber arranged at the distal catheter region of the catheter tube, a high pressure tube located in the lumen of the catheter tube for supplying a refrigerant to the gas expansion chamber, a low-pressure exhaust tube for discharging a gaseous refrigerant from the gas expansion chamber, and preferably a control unit for regulating the pressure and/ or the temperature of the refrigerant supply. Such cooling device is characterized in that the high-pressure tube comprises a proximal region, a transition region, and a distal region, the transition region being arranged in flow direction of the refrigerant between the proximal region and the distal region. The high-pressure tube further comprises at least one opening to the expansion chamber in the distal region. According to the invention the cooling device is designed in such a way that during use the refrigerant is passed through the proximal region of the high pressure tube in the liquid phase, the transition of the refrigerant into the gaseous phase takes place in the transition region and the refrigerant in the distal region is in gaseous phase where it exits through the at least one opening into the gas expansion chamber.

## Description

The invention relates to a cooling device for cooling a distal catheter region of a cryoablation catheter configured for ablation, a cryoablation device, a method for cooling a distal catheter region of a cryoablation catheter configured for ablation, and a method for therapeutic treatment of a human or animal body using a cryoablation catheter.

Cryoablation is used to alter the electrophysical properties of nerve cells by pre-cooling these nerve cells and then permanently inactivating them at temperatures as low as minus 75 degrees Celsius. As a result, they can neither generate nor transmit electrical impulses. This procedure is used, for example, to treat cardiac arrhythmias or hypertension. Special nerve cells for example in the heart tissue or in the tissue surrounding the renal arteries are ablated for this purpose. But cryoablation treatment is also suitable for sclerosing tumors.

Cryoablation by means of a cryoablation catheter is a minimally invasive procedure in which a corresponding catheter is inserted into a vessel of the human or animal body in order to ablate specific cells e.g. nerve cells at a suitable location. However, the insertion of the catheter in the body is risky, as it can lead to undesirable reactions, such as spasms. The relatively long retention time of the cryoablation catheter in the body during the treatment procedure is problematic, as the aforementioned risk increases with the duration of the treatment. It can be minimized by shorter treatment times. Decisive for the required retention time of the cryoablation catheter in the body is the time needed for the ablation catheter to cool down to the desired temperature for treatment and the duration of the cooling process for the cells to be ablated. This is particularly relevant for cryoablation catheters with smaller diameters, where it is especially advantageous to achieve temperatures as low as possible.

From US 2009/ 0 299 355 A1 and US 2015/ 0 265 334 A1, catheters for cryoablation are generally known.

The documents EP1398002B1 and WO2005095843A1 each describe cryoablation catheters with a cooling system, which is designed in such a way that the refrigerant is fed into a refrigeration/expansion chamber in a predominantly liquid state in which the phase transition of the refrigerant from liquid to gaseous takes place. In this process the refrigeration chamber is additionally cooled down by the transfer of the so-called latent heat from the environment to the boiling liquid, so that the efficiency of the cooling of the area designed for ablation body tissue is increased. In prior art documents the refrigerant is fed into the cooling/ expansion chamber in a predominantly liquid state and the phase transition takes place in the cooling/ expansion chamber.

In prior art N2O is often used as a coolant. The aggregate state of N2O for certain pressures and temperatures is shown in diagram 1 shown. The boiling curve is shown, i.e. at which pressure-temperature combinations the N2O liquid boils and changes into the gaseous phase.

The task of the invention is to provide an improved cooling device for cooling the ablation region/ surface of a cryoablation catheter or an improved cryoablation device and an improved cryoablation method associated therewith.

This task is solved by providing a cooling device for cooling a distal catheter region of a cryoablation catheter according to claim 1, a cryoablation device according to claim 8, a method for cooling a distal catheter region of a cryoablation catheter according to claim 9 and/or a method for therapeutic treatment of a human or animal body by means of a cryoablation catheter according to claim 11.

Surprisingly, it has been found that the cooling-efficiency can be further increased if the refrigerant already changes from liquid to the gaseous state shortly before it is introduced into the region formed for cooling/ expansion chamber. Instead of introducing the refrigerant in a liquid state into the region formed for cooling - the expansion chamber, e.g. formed as a balloon -, an increase in cooling capacity of more than 10% has been observed if the refrigerant is already in a gaseous state when introduced into the region formed for cooling (the expansion chamber). An additional temperature drop can be reached by balancing the pre-cooling of the N2O in the Cryo-System and the phase change and expansion of the N2O in the distal section of the Cryo-Catheter.

Therefore, the invention relates to a cooling device for cooling a distal catheter portion of a cryoablation catheter configured for ablation, the cooling device comprising a catheter tube formed with a lumen having a proximal catheter region and a distal catheter region, at least one gas expansion chamber arranged at the distal catheter region of the catheter tube, a high pressure tube located in the lumen of the catheter tube for supplying a refrigerant to the gas expansion chamber, a low-pressure exhaust tube for discharging a gaseous refrigerant from the gas expansion chamber, and preferably a control unit for regulating the pressure and/ or the temperature of the refrigerant supply. Such cooling device is characterized in that the high-pressure tube comprises a proximal region, a transition region, and a distal region, the transition region being arranged in flow direction of the refrigerant between the proximal region and the distal region.
- The high-pressure tube further comprises at least one opening to the expansion chamber in the distal region. According to the invention the cooling device is designed in such a way that during use the refrigerant is passed through the proximal region of the high pressure tube in the liquid phase,
- the transition of the refrigerant into the gaseous phase takes place in the transition region and
- the refrigerant in the distal region is in gaseous phase where it exits through the at least one opening into the gas expansion chamber.

Preferably the control unit for is adapted to control the temperature and/ or pressure of the refrigerant supply in order to make sure that the refrigerant exits into the expansion chamber in the gaseous state only.

There are different types of cryoablation catheters. For selective ablation, according to one embodiment the tip area of the cryoablation catheter is designed for ablation. If a larger area of nerve cells is to be ablated, a cryoablation catheter is suggested comprising side walls designed for ablation (for example, in the form of a balloon - so-called cryoballoon catheter). Such a device may be used for the treatment of electrical pulmonary vein isolation or for denervation of renal perivascular nerves. The present invention is suitable for use with all types of cryoablation catheters.

In a preferred embodiment of the invention, the distal portion of the cryoablation catheter designed for ablation is formed by at least one outer surface of the gas expansion chamber. For example, in a cryoballoon catheter, the cryoballoon preferably constitutes the gas expansion chamber.

According to the invention, a high-pressure tube is arranged in the lumen of the catheter, which is designed to supply the refrigerant in fluid to the gas expansion chamber.

Suitable refrigerants are substances which have a low boiling temperature at ambient pressure, preferably below -80°C at ambient temperature and pressure as well as a positive Joule-Thomson coefficient. N2O is particularly preferred. N20 can be absorbed by the blood and evaporated by the lungs in small amounts; This means small leakage in the system will not result directly in gas bubbles that can result in embolisms.

The refrigerant is supplied to the gas expansion chamber under pressure via the high pressure tube. The high pressure tube includes a proximal region, a transition region, and a distal region. In the proximal region/section, the refrigerant is kept under a pressure and temperature at which it is liquid. The refrigerant is kept in the transition section under a pressure and temperature that allows the transition from the liquid state to the gaseous state.

In a preferred embodiment, N2O is used as refrigerant.

The temperature-pressure combinations in which N2O is present in liquid or gaseous form can be taken from the diagram 1 shown.

Distal to the proximal region of the high-pressure tube is the transition region of the high-pressure tube. This transition region is designed in such a way that a transition from liquid to gaseous phase takes place.

According to the invention, the distal region of the high-pressure tube is arranged in flow direction of the refrigerant behind the transition region of the high-pressure tube. In the distal region of the high-pressure tube, the refrigerant is present in gaseous form.

The distal region of the high-pressure tube is equipped with at least one opening through which the gaseous refrigerant can escape into the gas expansion chamber.

If the cryoablation catheter is a cryoballoon catheter, the at least one opening is preferably arranged in the lateral side walls of the distal region of the high pressure tube so that the refrigerant gas can enter the gas expansion chamber through the side walls of the distal region of the high pressure tube.

Combinations of both embodiments are also possible, such that, for example, the tip region as well as the cryoballoon of a cryoablation catheter are configured for ablation.

The size of the at least one opening of the high pressure conduit is selected such that a sufficient pressure drop can be established between the distal region of the high pressure conduit and the gas expansion chamber.

Preferably, the transition region of the high pressure tube is located as close as possible to the at least one opening of the distal region of the high pressure tube.

Preferably, the side walls of the gas expansion chamber - at least partially - represent the distal catheter portion designed for ablation. For example, in a preferred embodiment, the cryoballoon of a cryoballoon catheter forms the gas expansion chamber.

In a preferred embodiment this is the balloon, in case of a cryoballoon catheter. The performance of the system can be tuned accordingly. In a preferred embodiment this can be done with the length of the transition region of the high pressure tube (first expansion), with the construction of the gas expansion chamber (second expansion), and the diameter and position of the expansion holes in the gas expansion chamber.

In another embodiment of the invention, the proximal region of the high pressure tube is closed at its distal end with a plate comprising at least one opening. Through this at least one opening, the refrigerant can enter the transition region.

The pressure and/ or the temperature of the refrigerant can also be controlled by varying the diameter of the high pressure tube in flow direction from the proximal to the transition and or to the distal region.

In another embodiment, the transition region is not arranged longitudinally downstream of the axis of the high-pressure tube, but radially surrounding it, thus enabling a more compact design.

In such a design the lumen of the transition region is formed by an outer element and an inner element. Preferably the inner element is the outer surface of the proximal region of the high-pressure tube (forming a core structure), and an outer element enclosing the inner element is formed by a hat, cap or jacket.

The Lumen of the transition region can be rotational symmetrical but preferably it is rotationally asymmetrical and even more preferably the lumen of the transition region is helically wound around the inner element/ core structure. Forming the lumen of the transition region, the inner wall of the hat, cap or jacket and or the outer wall of the inner element/ core structure can be grooved to form the e.g. helical lumen.

In this case the proximal region is preferably closed at its distal end, but it comprises at least one opening in the surrounding wall so that gaseous, partially gaseous or liquid refrigerant can escape through the hole(s) into the surrounding transition region.

Accordingly, as a preferred embodiment, the transition region (and preferably also the distal region of the high-pressure tube, as the transition region merges into the distal region) runs helix-shaped around the proximal region of the high-pressure tube.

The outer wall of the distal region according to this embodiment preferably comprises at least one opening. Preferably the refrigerant exits through the opening(s) into the gas expansion chamber, where it continues to expand and cool.

The gas expansion chamber surrounds the transition region and the distal region of the high-pressure tube. Preferably, the gas expansion chamber is formed by a cryoballoon.

Due to the helical lumen a pressure drop in the transition region takes place. As the refrigerant escapes through the openings into the surrounding chambers, a further pressure drop takes place. This allows the coolant to be successively cooled further until it reaches the desired temperature. Among other things, the dimensions of the helical groove and the size and/or arrangement of the openings can be used to shift the location of the phase transition and set a desired temperature and pressure. Accordingly, it is possible to run the device without a control unit for controlling the pressure and/ or the temperature if necessary.

It is well known that the refrigerant cools its surroundings during the phase transition from liquid to gas, as the latent heat required for the phase transition is absorbed from the surroundings. Surprisingly, better cooling of the catheter portion designed for ablation could be observed when the refrigerant is already in gaseous aggregation state before entering the gas expansion chamber. This could be explained by local decoupling of the latent heat cooling effect and the Joule - Thomson cooling effect, which leads to a reduction of the ambient temperature at enthalpic pressure reduction with the refrigerants used. A specific adjustment of the pressure in the different areas of the high pressure tube and the gas expansion chamber is essential for an optimal cooling of the refrigerant.

According to the invention, the cooling device for cooling a distal catheter region of a cryoablation catheter design for ablation comprises a low-pressure exhaust tube for discharging a gaseous refrigerant from the gas expansion chamber.

Preferably, the lumen of the gas expansion chamber is connected to the lumen of the catheter tube. In this embodiment, the low pressure exhaust tube is preferably formed by the lumen of the catheter tube itself, that is, the outer walls of the catheter tube also form the outer walls of the low pressure exhaust tube.

Preferably, the low pressure exhaust tube is connected in the proximal region to a device for adjusting the pressure. Such a device may be a vacuum device, in particular a vacuum pump.

In a preferred embodiment, the cooling device according to the invention comprises at least one pressure sensor. Preferably, this at least one pressure sensor may be arranged in at least one region of the high-pressure tube and/ or the low-pressure exhaust tube. Preferably, the at least one pressure sensor is arranged in the proximal region of the high-pressure tube.

In a further preferred embodiment, the cooling device according to the invention comprises at least one temperature sensor. Preferably, this at least one temperature sensor may be arranged in at least one region of the high-pressure tube, the low-pressure exhaust tube and/ or the gas expansion chamber. Preferably, the at least one temperature sensor is arranged in the gas expression chamber.

Also, preferably the at least one temperature sensor is a thermocouple.

In a particular embodiment of the invention, the control unit for regulating the pressure of the refrigerant supply is connected to the at least one temperature sensor.

In another embodiment of the invention, the control unit for regulating the pressure of the refrigerant supply is connected to the at least one pressure sensor. Preferably, the control of the pressure of the refrigerant supply is based on the measured pressure and/or as a function of a measured pressure curve and/ or as a function of the measured temperature. Controlling on pressure and temperature is crucial for the function of the system.

In a lab set-up the performance of the different nozzle configurations can be tested. The control of the final product can be set accordingly. In some set-ups the distance between the control unit and catheter might be more than 4 m, preferably max. 5 m. For example, for the performance of ablations under MRI (magnetic resonance imaging). Then the set-up of the system needs to be modified accordingly. The aim will always be to have the highest possible pressure in the high pressure tube and the lowest possible temperature of the liquid N2O.

According to a further embodiment of the invention, the at least one temperature sensor is connected to the control unit. Based on the measured temperature or the change in the measured temperature the pressure of the refrigerant supply is regulated by the control unit.

In this embodiment, the regulation of the pressure of the refrigerant supply is preferably performed as a function of the measured temperature and/or as a function of a measured temperature profile.

Further preferably, the control unit according to the invention is connected to at least one device for adjusting the pressure to control the supply of the refrigerant according to the measured temperature and/or as a function of a measured temperature curve and/or as a function of a measured pressure or pressure curve. In a preferred embodiment of the invention, the at least one device for adjusting the pressure is a proportional valve and/ or a device for adjusting a negative pressure, preferably a vacuum device.

In one embodiment of the invention, the pressure of the refrigerant is controlled by controlling the supply of the refrigerant, preferably by means of a proportional valve. Control of the pressure by means of a vacuum device is also conceivable.

In another preferred embodiment the inflowing refrigerant might be controlled by temperature. Preferably the temperature is controlled and regulated by the control unit. As an example, the high-pressure inflowing refrigerant can be heated, resulting in faster transfer into the gaseous state.

Accordingly, the device according to the invention preferably further comprises a heating device/ heater. The heater can be arranged anywhere in the device, preferably in the in the high-pressure tube, more preferred in proximity to or in the transition region. Each type of heater known in the state of the art may be used.

The invention further relates to a cryoablation device comprising the cooling device according to the invention, a source for the refrigerant, a heat exchanger/cooling device for cooling and if necessary, liquefying the refrigerant, and a vacuum device for extracting the refrigerant gas from the gas expansion chamber.

High-pressure gas cylinders are preferably suitable as the source for the refrigerant. The heat exchanger is arranged between the source for the refrigerant and the high-pressure tube. The heat exchanger serves to cool down the gaseous refrigerant, whereby it is liquefied, and pre-cooled.

It then enters the high-pressure tube in the liquid state.

Preferably, a proportional valve is arranged between the source of refrigerant and the heat exchanger.

Preferably, the vacuum device for extracting the gaseous refrigerant is arranged at the low pressure exhaust tube.

The invention also relates to a method for cooling a distal catheter region of a cryoablation catheter configured for ablation, comprising the steps of: introducing a precooled refrigerant into the high-pressure tube of a cryoablation catheter having a temperature T1, preferably measuring at least one temperature T2 in the gas expansion chamber, possibly controlling the temperature T2 by means of a device for adjusting the pressure and / or the temperature of the refrigerant supply in such a way that the refrigerant changes from a liquid to a gaseous aggregate state prior to exiting through the at least one opening in the distal region of the high-pressure tube into a gas expansion chamber. A desired temperatureT3 is set in the distal catheter portion of the cryoablation catheter on the outer surface of the gas expansion chamber designed for ablation.

According to the invention, the measurement of the temperatures can be continuous or discrete. Alternatively, or additionally, temperature measurements and/or pressure measurements can be made at further locations of the cryoablation device.

By means of the control unit, the measured data is evaluated and the pressure or pressures and/ or temperature in the cryoablation device are adjusted in such a way that the refrigerant flows through the proximal region of the high-pressure tube in a liquid phase, a phase transition of the refrigerant from liquid to gaseous takes place in the transition region, and the refrigerant in the distal region is present in gaseous phase before exiting into the expansion chamber.

Due to the high thermal conductivity and/or low wall thickness of the gas expansion chamber of the distal catheter portion of the cryoablation catheter designed for ablation, preferably the temperature T3 differs only minimally from the temperature T2 prevailing in the gas expansion chamber.

In one embodiment of the invention, when the temperature T2 in the gas expansion chamber drops, the supply of the refrigerant is reduced and when the temperature T2 in the gas expansion chamber rises, an increase in pressure in the supply tube is effected by increasing the supply of the refrigerant or by regulating the temperature of the refrigerant. This might be achieved by precooling or cooling of the refrigerant.

The invention further relates to a method for therapeutic treatment of a human or animal body comprising the steps of: introducing a cryoablation catheter into a vessel of the human or animal body, introducing a refrigerant into a supply tube to a heat exchanger, cooling the refrigerant to a temperature T1, introducing the precooled and liquid refrigerant into a high-pressure tube of a cryoablation catheter, measuring at least one temperature T2 in a gas expansion chamber, and optionally regulating the temperature T2 by means of a control unit for regulating the pressure and or temperature of the refrigerant supply in such a way that the refrigerant changes from a liquid to a gaseous aggregate state prior to contact with at least one opening in the distal region of the high-pressure tube and subsequently emitting the refrigerant as a gas through the at least one opening into the gas expansion chamber which eventually sets a temperature T3 on the outer surface of the gas expansion chamber, on the distal catheter portion of the cryoablation catheter designed for ablation.

In one preferred embodiment of the invention the method according to the invention is characterized in that: when the temperature T2 in the gas expansion chamber decreases, a decrease in the pressure of the supply of the refrigerant is effected, and when the temperature T2 in the gas expansion chamber increases, an increase in the pressure of the supply of the refrigerant is effected.

As an alternative to the temperature T2, the temperature T3 can also be measured and used as a parameter.

Further details can be obtained from the description of the figures, which represent preferred embodiments of the invention showing:
Fig. 1 a side view of a high-pressure tube;
Fig. 2 a side view of a high-pressure tube with a gas expansion chamber;
Fig. 3 a schematic view of a cryoablation device;
Fig. 4 a vapor pressure curve of N2O;
Fig. 5 cross section of the distal region of a high pressure tube comprising a double chamber nozzle;
Fig. 6 clear cut side view of the distal region of a high pressure tube comprising a double chamber nozzle;
Fig. 7 schematic view of the cooling device comprising a heater;
Fig. 8 schematic view of the cooling device comprising a pressure release valve.

It is noted that the presented pictures are not to scale.

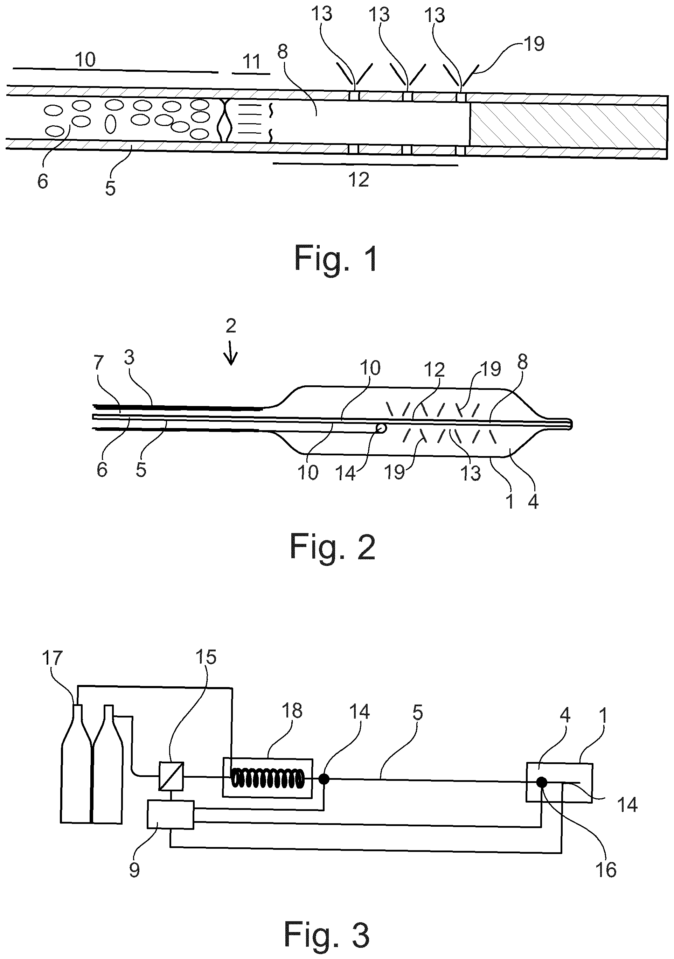

Figure 1 shows a longitudinal cross section view of a high pressure tube 5 as part of a catheter 2 with a proximal region 10 of the high pressure tube 5, a transition region 11 of the high pressure tube 5 and a distal region 12 of the high pressure tube 5. The high pressure tube 5 is embedded in the lumen of the catheter tube 3. The high-pressure tube 5 is closed at the distal end so that no refrigerant exits the tube here. The liquid refrigerant 6 flows through the proximal region 10 of the high-pressure tube 5. In the transition region 11 of the high-pressure tube 5, the phase transition of the liquid refrigerant 6 from liquid to gaseous takes place. Subsequently, the gaseous refrigerant 8 flows into the distal region 12 of the high-pressure tube 5, where it exits laterally from the distal region 12 of the high-pressure tube 5 through the at least one opening 13. The escaping refrigerant gas 19 expands as it exits. Thus, a twofold cooling effect takes place, once by the phase transition from liquid to gaseous in the transition region 11 of the high-pressure tube 5 and another time when it escapes through the at least one opening 13 of the high-pressure tube 5 due to the associated expansion.

It is essential that the phase transition of the refrigerant occurs shortly before the further expansion of the gaseous refrigerant. Therefore, the transition region 11 of the high pressure tube 5 is arranged as close as possible to the distal region 12 of the high pressure tube 5 having at least one opening 13.

Figure 2 shows a longitudinal section through a high-pressure tube 5 with a gas expansion chamber 4. Here, the high-pressure tube 5 is arranged in the lumen of the catheter tube 3. Figure 2 additionally shows the proximal region 10 of the high pressure tube 5, the transition region 11 of the high pressure tube 5 and the distal region 12 of the high pressure tube 5.

In addition, a low pressure exhaust tube 7 is shown in the catheter tube 3, which in this embodiment is formed by the lumen of the catheter tube 3 itself.

In the illustrated embodiment of the invention, the entire distal region 12 of the high pressure tube 5, the transition region 11 of the high pressure tube 5 and a portion of the proximal region 10 are surrounded by the gas expansion chamber 4. In this embodiment, the walls of the gas expansion chamber 4 form the distal catheter portion 1 of the cryoablation catheter which is designed for ablation. The lumen of the gas expansion chamber 4 is connected, for example welded or glued, to the lumen of the catheter tube 3, so that the refrigerant gas 19 emerging from the at least one opening 13 can be discharged via the low-pressure exhaust tube 7 after its expansion in the gas expansion chamber 4.

By means of a temperature sensor 14 in the gas expansion chamber, the temperature T2 in the gas expansion chamber can be measured and used as a controlled parameter/variable. By means of a regulation of the pressure and/ or temperature of the refrigerant supply and/or the vacuum pump, the temperature T2 can be adjusted. Alternatively, a temperature T3 of the distal catheter portion of the cryoablation catheter designed for ablation (the outer surface of the gas expansion chamber) can also serve as a controlled variable. In the present embodiment, the two temperatures hardly differ, since the distal catheter portion of the cryoablation catheter designed for ablation is formed by the thin side walls of the gas expansion chamber 4.

Figure 3 shows a schematic view of the cryoablation device according to the invention. In this embodiment, the distal catheter portion of the cryoablation catheter designed for ablation is formed by the side walls of the gas expansion chamber 4. In addition to the temperature sensor 14, an (optional) pressure sensor 16, arranged in the gas expansion chamber 4, is also shown. Both sensors are connected to the control unit for regulating the pressure of the refrigerant supply 9, which in turn is connected to a device for regulating the pressure, in this case a proportional valve 15. Another (optional) pressure sensor 14 is located in the proximal region of the high pressure tube 5 where the refrigerant exits the heat exchanger 18 and is also connected to the control unit for regulating the pressure of the refrigerant supply 9, which regulates the temperature of the refrigerant in the cryoablation device by varying the supply of the refrigerant via the proportional valve 15. From a source of refrigerant 17, the refrigerant is supplied to the cryoablation device.

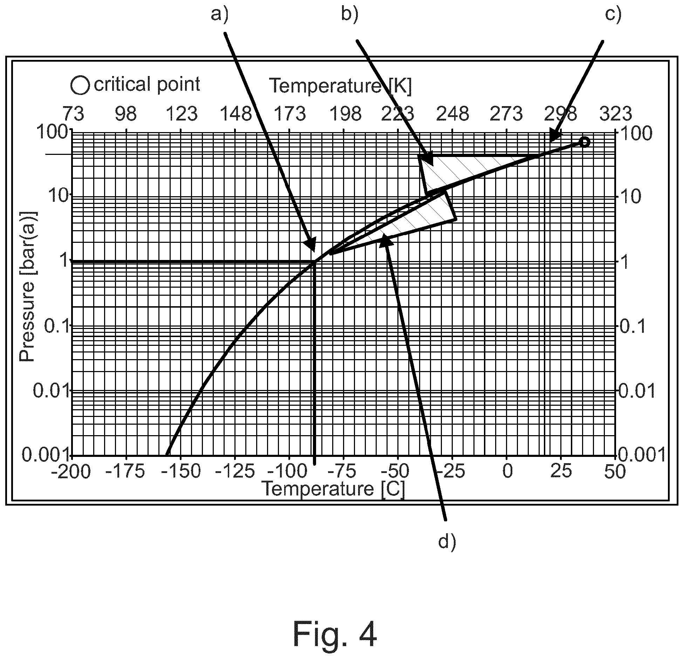

Figure 4 shows a vapor pressure curve of N2O. The boiling temperatures for certain pressures are shown, the states of aggregation can be read of the N2O at different pressure/temperature combinations. Possible preferred temperature-pressure ranges for the solution according to the invention are shown by way of example, a) shows optimal temperature-pressure values for the gaseous NO2 in the gas expansion chamber 4. In the gas expansion chamber 4, the pressure should be as close as possible to atmospheric pressure so that the minimum temperature of -88,6°C could be reached.

b) shows a possible temperature-pressure range for the liquid N2O in the proximal region 10 of the high-pressure tube 5.

By pre-cooling the liquid N2O, (cooling) energy is brought in the N2O that is used to increase the cooling performance of the device. The area left from the graph is preferred until the liquid N2O enters the transition region 11 of the high pressure tube 5.

c) marks the boiling point at room temperature (20°C with 48bar).

d) shows a possible temperature-pressure range for the N2O in the transition region 11 of the high-pressure tube 5.

During the phase change in the transition region 11 of the high-pressure tube 5 the temperature drop in the system is extreme. To achieve the lowest possible temperature the balance between phase change and expansion can be regulated by the proportional valve. The ideal situation is to keep the phase change in the transition region 11 separate from the expansion in the gas expansion chamber 4.

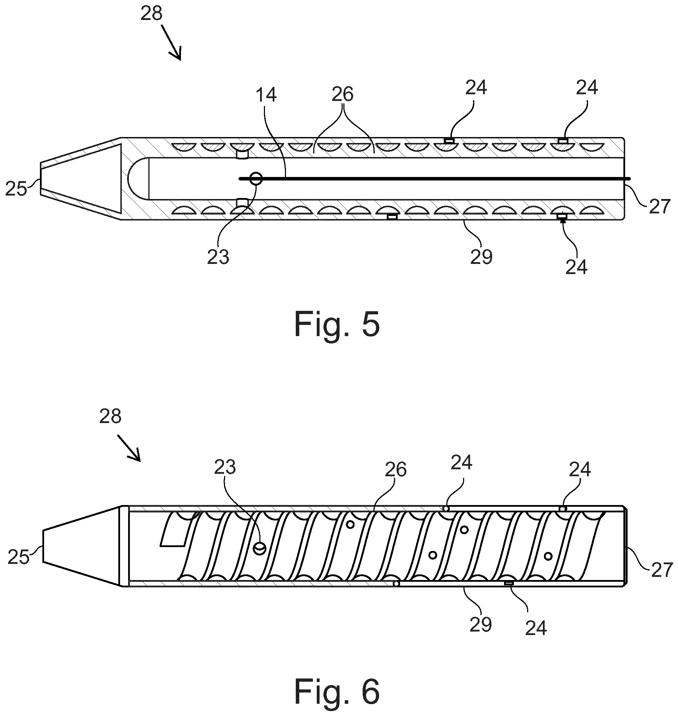

Fig. 5 shows a cross sectional view of the distal region of a high pressure tube 5 comprising a double chamber nozzle 28. Gaseous or liquid refrigerant enters the inner chamber 30 of the double chamber nozzle 28 through the entrance of the inner chamber 27 and flows through the at least one opening 23 of the inner wall of the double chamber nozzle 28 into the outer chamber of the double chamber nozzle 28. In the outer chamber of the double chamber nozzle 28, which is formed by helix shaped grooves 26 the gaseous or liquid refrigerant flows along the helical structure (helix or double helix pattern) 26 and enters the gas expansion chamber 4 (not shown) via the at least one opening 24 in the outer wall 29 of the outer chamber of the double chamber nozzle 28. Multiple expansions of the gaseous refrigerant due to pressure drops caused by the structure of the double chamber nozzle 28 take place. The gaseous refrigerant continues to cool until it exits into the gas expansion chamber 4 (not shown); preferably a cryoballoon 32 (not shown). According to the invention, the refrigerant enters the gas expansion chamber 4 in the gaseous state. If the refrigerant enters the nozzle in liquid state, the outer chamber can be considered partly as transitional region if the refrigerant enters the outer chamber in (partly) liquid state and it is considered as distal region if or once the refrigerant has transferred into the gaseous state. It should be emphasized however, that the double chamber nozzle comprising an inner chamber and an outer chamber as described above and below including the holes and grooves and being adapted to be integrated in a cryoablation device, is an independent inventive aspect irrespective of whether the refrigerant passes through the nozzle and exits it in a liquid or gaseous state. If - on the other hand - the person skilled in the art wishes to provide a nozzle that provokes a phase change due to pressure drop and make sure that a refrigerant exits the nozzle in a gaseous state only it is easy for him or her to adapt the dimensions of the nozzle accordingly to reach this goal for any given temperature and pressure of the refrigerant entering the nozzle.

Figure 6 shows a clear cut side view of the distal region of a high pressure tube shown in figure 5 comprising the double chamber nozzle 28. The helix pattern/groove 26 is shown. The refrigerant flows between the grooves 26 and the outer wall 29 of the nozzle, formed as a cylindrical tube/ cap. The refrigerant is cooled down due to expansion while travelling down the helix pattern 26. According to an embodiment it can be provided that the height of the helical passage increases in the direction of flow. The tip 25 of the nozzle is preferably glued to the cryoballoon 32. It has a conical shape with a rounded tip. In this variant it is closed, no gas enters the cryoballoon 32 or the gas expansion chamber 4 respectively via the tip 25 of the nozzle.

Openings 23, 24 of the outer and the inner wall of the double chamber nozzle 28 are shown, wherein the inner wall of the outer chamber of the double chamber nozzle 28 at the same time is the outer wall of the inner chamber of the double chamber nozzle 28.

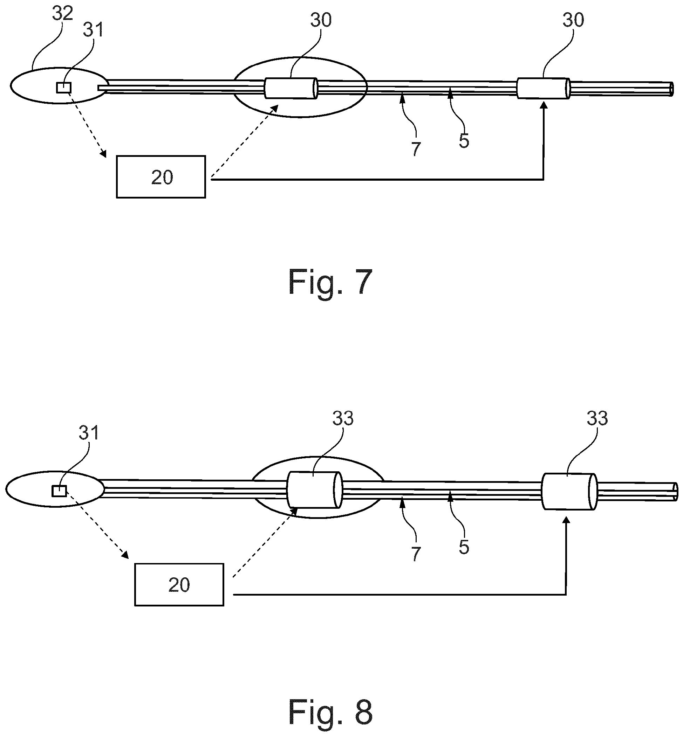

Figure 7 shows a schematic view of the cooling device comprising a heater 30. Via a sensor 31, which might be a pressure or a temperature sensor, in this embodiment arranged inside the cryoballoon 32, temperature and/or pressure of the refrigerant can be measured. Relating to the measured parameter a heater 30 is regulated by the control unit 20. A heater 30 can be positioned at any location in the high-pressure tube 5 between the heat exchanger 18 and the transition region 11 or even inside the transition region 11.

If the pressure and/or the temperature is not appropriate to achieve the desired final cooling temperature of the refrigerant, it can be regulated by regulating the heater 30 via the control unit 20. Changing of the position of the phase transition has a large influence on the final cooling temperature. Small changes in temperature and therefore the use of small heaters 30 are sufficient to reach the desired goal.

Figure 8 shows a schematic view of the cooling device comprising a pressure release valve 33. The principle underlying this arrangement for regulating the pressure and thus also the temperature of the refrigerant is the same as that shown for Figure 7. The sensor 31 can again be either a pressure or a temperature sensor. Regulation takes place via a pressure release valve 33, which can reduce the inflowing pressure by releasing some of the high-pressure coolant directly into the exhaust path 7. Preferably, this valve 33 is located between the heat exchanger 18) and the distal region 12 of the high pressure tube 5.

For example, the pressure release valve can also be located inside the catheter handle, or in the console. As another embodiment a pressure release valve 33 as well as a heater 30 can be provided.

A preferred embodiment of the invention should be exemplified in the following:
In this preferred embodiment, the pressure of the refrigerant in the proximal region of the high pressure tube is in the range between 44 bar and 52 bar, preferably 44,5 bar -51 bar, even more preferably between 45 bar and 50 bar.

It is aimed to keep the pressure as close as possible to 50 bar. When the pressure stays high, 50bar, and the temperature decreases the thermodynamic cooling capacity of the N2O increases. This increases the freezing performance/ cooling power of the catheter.

The heat exchanger serves to cool down the refrigerant, whereby it is (further) liquefied, and pre-cooled.

The temperature in the refrigerant supply can be between 15°C to -40°C, preferably between 13°C and -30°C even more preferably between 12°C and -20°C and even more preferably between 10°C to -10°C.

The pressure of the N2O refrigerant might be by entering the transition region in a preferred range between 45 bar - 50 bar, preferably 46 bar - 50 bar, more preferably 47 bar - 50 bar. In the transition region the pressure is decreasing.

At the entrance of the transition region, the temperature might be preferably between -8 and -40°C, more preferably between -10°C and -35°C and even more preferably between -10°C and -30°C.

At the start of the ablation process the temperature of the liquid N2O at the entrance of the transition region might be a bit higher between -8°C and be -12°C, since a low temperature cannot be reached from the start. During the ablation process the temperature at the entrance of the transition region will decrease and might preferably reach until -40°C.

In the gas expansion chamber, the pressure might be preferably 1 bar - 5 bar, more preferred 1 bar - 3 bar, even more preferred 1 bar - 2 bar. Due to the expansion of the gaseous refrigerant into the gas expansion chamber a temperature of -88,6°C can be reached.

## Claims

1. Cooling device for cooling a distal catheter portion of a cryoablation catheter adapted for ablation comprising
- a catheter tube formed with a lumen and having a proximal catheter region and a distal catheter region;
- at least one gas expansion chamber arranged at the distal catheter region of the catheter tube; and
- a high pressure tube arranged at least partly in the lumen of the catheter tube for supplying a refrigerant to the gas expansion chamber;
- a low-pressure exhaust tube for exhausting a gaseous refrigerant from the gas expansion chamber;
- a control unit for regulating the pressure and/or temperature of the refrigerant in the high pressure tube;
**characterized in that**
- the high pressure tube further comprises a proximal, a transition and a distal region, the transition region being arranged in flow direction of the refrigerant between the proximal and the distal region, and wherein the high pressure tube has at least one opening to the expansion chamber in the distal region, and
- the control unit is configured to control the pressure and/or temperature of the refrigerant in such a way, that during use the refrigerant is passed through the proximal region of the high-pressure tube in liquid phase, a phase transition of the refrigerant from liquid to gaseous takes place in the transition region and the refrigerant is present in the distal region in gaseous phase where it emerges through the at least one opening into the gas expansion chamber.

2. Cooling device according to any one of the preceding claims, comprising at least one temperature sensor and / or at least one pressure sensor, arranged in the gas expansion chamber and/or in the high-pressure tube, which is connected to the control unit for regulating the pressure and/ or temperature of the refrigerant supply, wherein the regulation of the pressure and or the temperature of the refrigerant takes place as a function of the measured temperature and/or as a function of a measured temperature profile and/or as a function of the measured pressure.

3. Cooling device according to any one of the preceding claims, wherein the control unit is provided with at least one device for adjusting the pressure in order to control the supply of the refrigerant in accordance with the measured temperature and/or in dependence on a measured temperature profile and/ or the measured pressure.

4. Cooling device according to claim 3, wherein the at least one device for adjusting the pressure comprises a proportional valve.

5. Cooling device according to any one of the preceding claims comprising a pressure-release valve.

6. Cooling device according to any one of the preceding claims comprising a heater.

7. Cooling device according to one of the preceding claims wherein the at least one temperature sensor is a thermocouple.

8. Cooling device according to any of the preceding claims comprising at least one pressure sensor.

9. Cryoablation device comprising
- a cooling device according to claim 1,
- a source for the refrigerant;
- a heat exchanger for cooling and liquefying the refrigerant;
- a vacuum device, to exhaust the refrigerant gas exiting into the gas expansion chamber.

10. Method of cooling a distal catheter region of a cryoablation catheter adapted for ablation comprising the steps of:
- introducing a precooled refrigerant into the high-pressure tube of a cryoablation catheter at a temperature T1;
- measurement of at least one temperature T2 in the gas expansion chamber;
- if necessary, regulating the temperature T2 by means of a device for adjusting the pressure of the refrigerant supply in such a way that the refrigerant changes from a liquid to a gaseous aggregate state prior to contact with at least one opening in the distal region of the high-pressure tube and subsequently flows as a gas through the at least one opening into a gas expansion chamber and a desired temperature T3 is set on the surface of the distal catheter portion of the cryoablation catheter designed for ablation.

11. Method according to claim 10, **characterized in that** when the temperature T2 in the gas expansion chamber decreases, a decrease in the pressure is brought about by decreasing the supply of the refrigerant and that when the temperature T2 in the gas expansion chamber increases, an increase in the pressure is brought about by increasing the supply of the refrigerant.

12. Method for therapeutic treatment of a human or animal body comprising the steps of:
- inserting a cryoablation catheter into a vessel of the human or animal body;
- introducing a refrigerant into a supply tube to a heat exchanger;
- cooling the refrigerant to a temperature T1;
- introduction of the pre-cooled and liquid refrigerant into a high-pressure tube of a cryoablation catheter;
- measuring of at least one temperature T2 in a gas expansion chamber;
- if necessary, regulating the temperature T2 by means of a control unit for regulating the pressure of the refrigerant supply in such a way that the refrigerant changes from a liquid to a gaseous aggregate state before coming into contact with at least one opening in the distal region of the high-pressure tube and then flows as a gas through the at least one opening into the gas expansion chamber and a desired temperature T3 is set on the surface of the distal catheter portion of the cryoablation catheter designed for ablation.

13. The method according to claim 12, **characterized in that** when the temperature T2 in the gas expansion chamber drops, a drop in pressure is effected by reducing the supply of the refrigerant and that when the temperature T2 in the gas expansion chamber rises, an increase in pressure is effected by increasing the supply of the refrigerant.
